# EUROPEAN PATENT SPECIFICATION

(11) **EP 1 962 798 B1**
(45) Date of publication and mention of the grant of the patent: **12.09.2018**
(21) Application number: 06846243.1
(22) Date of filing: 07.11.2006
(51) Int. Cl.: A61K 9/00

(54) **INJECTABLE FORMULATION CAPABLE OF FORMING A DRUG-RELEASING DEVICE**
INJIZIERBARE FORMULIERUNG MIT FUNKTION ZUR FORMUNG EINER WIRKSTOFFFREISETZUNGSVORRICHTUNG
FORMULATION INJECTABLE POUVANT FORMER UN DISPOSITIF D'ADMINISTRATION DE MEDICAMENT

(30) Priority: 14.11.2005 US 273247
(43) Date of publication of application: 03.09.2008
(73) Proprietor: Medtronic Xomed, Inc., Jacksonville, FL 32216-0980 (US)
(72) Inventor: TIJSMA, Edze J., NL-6224 LD Maastricht (NL); HISSONG, James B., Jacksonville, Florida 32224 (US); GONZALEZ, Maria N., NL-6211 SW Maastricht (NL)
(74) Representative: Dehns
(86) International application number: PCT/US2006/060602
(87) International publication number: WO 2007/059390

(56) References cited:
- EP-A- 1 374 856
- WO-A-00/21510
- WO-A-94/05330
- WO-A-99/27905
- WO-A-2004/024187
- WO-A-2005/089670
- WO-A2-2006/034467
- FR-A- 2 710 529

## Description

### FIELD OF THE INVENTION

The present invention is in the field of medical devices and relates to devices for releasing active substances directly to damaged tissues in cavities of a vertebrate's skull, such as the paranasal sinus of a human patient. The invention further relates to formulations and methods for the preparation of these formulations and devices and to their use as medicaments in treatment and prevention of disease in said cavities. It is an attribute of the medicaments that they are easy to deploy. The invention also relates to methods of treating sinus disease, and especially sinusitis.

### BACKGROUND OF THE INVENTION

Sinusitis, the inflammation of the mucosal tissues in the paranasal sinuses, is a common disease that affects humans throughout their lives. The symptoms of sinusitis include headache, nasal drainage (rhinorrhea,) jaw and/or teeth sensitivity, swelling around the eyes, nasal congestion and loss of smell.

In many cases sinusitis is caused by viral infection of the upper respiratory system, but it may also be the result of allergies, medication or structural abnormalities in the (para)nasal cavities and nasal passageways. Sinusitis exists in different forms, the chronic forms being classified as chronic rhinosinusitis (CRS) and nasal polyposis (NP).

The paranasal sinuses are present in four left and right pairs: the frontal sinuses positioned over the eyes in the brow area, the maxillary sinuses inside each cheekbone, the ethmoid sinuses just behind the bridge of the nose and between the eyes, and the sphenoid sinuses behind the ethmoids in the upper region of the nose and behind the eyes. These sinuses constitute air-filled cavities in the bones of the skull and are connected to the nasal passageways by small openings (ostia), which allow passage of air to and from the sinus and the drainage of mucous produced by mucosal tissue that lines the sinus walls.

Inflammation of these tissues may lead to blockage of the passageways and the stagnation of mucous may result in bacterial or even fungal infection of the sinus cavities. When symptoms of sinusitis persist and are not responsive to nasal medications, bacterial infection is suspected and antibiotic therapy is prescribed. When all pharmaceutical treatments of sinusitis fail, severe acute sinusitis, CRS and NP may require sinus surgery, which involves opening of sinuses and removal of pathological mucosal tissue.

As an endoscopic technique, Functional Endoscopic Sinus Surgery (FESS) is now the preferred procedure for sinus surgery and for the medical management of CRS and NP. Although the functional results of FESS are satisfactory in the majority of cases, wound healing of the mucosal tissues after FESS is poor in about 20% of patients. This poor healing is associated with abnormal scarring, super-infection, and fibrosis formation, and these complications may in turn lead to recurrence of symptoms and the necessity of revision surgery.

With respect to postoperative wound treatment, endoscopic surgical techniques have made the conventional extensive nasal packing obsolete. Currently, postoperative nasal packing is not so much a hemostatic measure but is rather employed as a spacer to stent the (para)nasal cavity. The therapeutic modality or purpose of the spacer or stent is to provide postoperative drainage of the sinus and to prevent postoperative blockage of the outflow tract. The stent is shaped to fit and be retained in a particular part of the (para)nasal cavity and to leave room for airflow. This minimal packing may consist of a biodegradable or absorbable packing material. If not absorbable, the stent is commonly removed 1 to 4 days after the operation. Such stents however do not comprise any specific medication for wound healing.

The problems that exist in treating damaged mucosa or other diseases in the paranasal sinus cavities also exist for other cavities of the vertebrate skull, such as the inner ear.

Therefore, there is at present still a need for a device that is adapted for a more easy deployment in difficult-to-reach mucosa-lined cavities of the skull of a vertebrate individual, such as the (para)nasal cavities of a human patient, and which device is adapted for controlled release of active substances that can aid in treatment, and/or improve the healing process, in particular the wound healing after sinus surgery. There is at present still a need for improved devices such as stents suitable for use in a surgical environment including FESS, especially under minimally invasive surgical procedures.

WO 2005/089670 describes pharmaceutical compositions that are liquid during administration and which form a gel or semi-solid bolus of a hydrophobic polymer in situ in the sinus.

WO 94/05330 describes a nasal pharmaceutical product in sprayable form which contains a carboxyl-containing polymer and a cross-linking agent. On contact with the mucous linings of the nasal cavity it increases in viscosity to form a gel.

### SUMMARY OF THE INVENTION

The present inventors have found an injectable and hardenable formulation that upon hardening is capable of active substance release.

One aspect of the present invention provides an injectable formulation for use in treatment of a disease in a (para)nasal cavity in an individual's skull, the formulation comprising an active substance and a formable material designed to adapt and conform to the shape of at least a part of said cavity when engaged therewith, wherein said material has a formable shape or low viscosity and upon engagement with at least a part of said cavity is hardenable to provide a device with a permanent shape or higher viscosity, wherein said device when hardened provides controlled release over a predetermined period of time of from 1 day to 12 months of a therapeutically effective amount of said active substance, and said formable material:
a) is an injectable reactive self-forming gel system hardenable by photopolymerization or electrophilic-nucleophilic reaction, or an in-situ forming gel system hardenable by cooling or stereocomplexation, or
b) is in the form of polymer microspheres dispersed in a gel, or
c) comprises a water-soluble polymer which when engaged with at least a part of said cavity forms a hydrogel generated by crosslinking of the water-soluble polymer.

Another aspect of the present invention provides an injectable formulation comprising an active substance and a formable material designed to adapt and conform to the shape of at least a part of a cavity of an individual's skull when engaged therewith, wherein said active substance is selected from the group consisting of matrix metalloproteinase inhibitors, COX-2 inhibitors, ACE-inhibitors and ARBs, Chymase inhibitors, therapeutic polymers and combinations thereof, wherein said material has a formable shape or low viscosity and upon engagement with at least a part of said cavity is hardenable to provide a device with a permanent shape or higher viscosity, wherein said device when hardened provides controlled release over a predetermined period of time of from 1 day to 12 months of a therapeutically effective amount of said active substance, and said formable material:
a) is an injectable reactive self-forming gel system hardenable by photopolymerization or electrophilic-nucleophilic reaction, or an in-situ forming gel system hardenable by cooling or stereocomplexation, or
b) is in the form of polymer microspheres dispersed in a gel, or
c) comprises a water-soluble polymer which when engaged with at least a part of said cavity forms a hydrogel generated by crosslinking of the water-soluble polymer.

Certain embodiments of the present invention are indicated for use in any cavity of the vertebrate skull that is lined with mucosa, in particular such cavities that are air-filled, preferably the paranasal sinuses (frontal, maxillary, ethmoid and/or sphenoid) and/or nasal passageway.

In another embodiment of the above formulations, the formable and hardenable material is a biodegradable material.

Any substance may be used as active substance in a device of certain embodiments of the invention. In another embodiment of the invention, the active substance is selected from the group consisting of MMP inhibitors, cyclooxygenase-2 (COX-2) inhibitors, angiotensin convertin enzyme (ACE)-inhibitors and angiotensin receptor blockers (ARBs), Chymase inhibitors, therapeutic polymers and combinations thereof.

In accordance with an embodiment of the invention, one or more of the major classes of MMP inhibitor compounds may be used, in particular one or more compounds selected from the group consisting of hydroxamic acids, carboxylic acids, thiols, phosphinic acids, and tetracyclines. In a further embodiment, preferred MMP inhibitors include inhibitors selected from the group consisting of *N*-biphenyl sulfonyl-phenylalanine hydroxamic acid; amines, amino acid derivatives and low molecular weight peptides containing an amide-bound oxal hydroxamic acid moiety; benzodiazepine; acyclic succinic acid-based compounds; oleic acid; grape seed extract (GSE); cerivastatin; thiol compound MAG-283; tetracycline derivatives, such as tetracycline, doxycycline, and minocycline. In a further embodiment, the MMP inhibitors are doxycycline and dexamethasone.

In a further embodiment, said formulation further comprises at least one pharmaceutical agent involved in remodelling processes.

In another aspect, disclosed herein is an active substance-releasing device obtainable by hardening the formulation of the present invention.

Also disclosed herein is a method for treatment of a disease or damaged mucosal tissue in a cavity of an individual's skull, said method comprising: a) introducing into the cavity of an individual's skull an injectable formulation as herein described comprising an active substance and a formable material designed to adapt and conform to the shape of at least a part of said cavity when engaged therewith, wherein said material is hardenable upon engagement with at least a part of said cavity to provide a device with a permanent shape, and wherein said device is capable of releasing a therapeutically effective amount of said active substance when hardened, and b) hardening said formulation.

In one embodiment of such a method, said cavity of an individual's skull is a paranasal sinus, the nasal passageway and/or the middle ear cavity, preferably a paranasal sinus of a human.

In another embodiment of such a method, said mucosal tissue is selected from the group consisting of ethmoid sinus mucosal tissue, maxillary sinus mucosal tissue, sphenoid sinus mucosal tissue, frontal sinus mucosal tissue and their ostia and combinations thereof.

The method of treatment in one embodiment of the present disclosure relates to the application of the formulations and devices as above described, and the formulation may be used in such a method in any of the embodiments stated above.

### DETAILED DESCRIPTION OF THE INVENTION

### A. Definitions

The term "cavity of an individual's skull" refers to the air-filled, mucosa-lined cavities located within the dense craniofacial bones surrounding the nose as well as to the air filled cavities in the petrous temporal bone of a vertebrate, and includes the paranasal sinuses as well as other difficult-to-reach skull cavities such as the middle ear and pharyngotympanic tube and the air cells of the mastoid process.

The terms "paranasal sinus", "nasal sinus" and "sinus" are used interchangeably herein and indicate an air-filled cavity in the bones of the skull connected to the nasal passageways by small openings (ostia), which allow passage of air to and from the sinus and the drainage of mucous produced by mucosal tissue that lines the sinus walls. The sinuses are present in four left and right pairs: the frontal sinuses positioned over the eyes in the brow area, the maxillary sinuses inside each cheekbone, the ethmoid sinuses just behind the bridge of the nose and between the eyes, and the sphenoid sinuses behind the ethmoids in the upper region of the nose and behind the eyes. In relation to acute sinusitis, CRS and NP the ethmoidal cleft and frontal sinus are in particular indicated for treatment.

The term "nasal passageway" refers to the passageway that extends from the nasal openings to the choanae, the openings in the roof or soft palate region of the mouth that connect the nasal cavity to the pharynx.

The term "(para)nasal cavity" includes both the paranasal sinuses and nasal passageways.

The term "mucosal tissue" includes mucous producing tissue of both the paranasal sinus cavities, nasal passageways and middle ear cavities, including the pharyngotympanic tube.

The term "injectable formulation" refers to an injectable composition, such as in the form of a powder or, more preferably, in the form of a fluid, semi-solid or gel-like substance. The term "injectable" means that the formulation is engageable and/or insertable into or onto a desired location of the body of an individual or patient, e.g. in a cavity of an individual's skull, preferably by being able to flow under the application of a sufficient pressure, in particular through a needle or catheter or other suitable applicator for the application of powders or, more preferably, fluids, semi-solids or gel-like substances.

The term "formulation" means, in its broadest sense, a materials mixture or composition wherein the active substance is formulated, mixed, added, dissolved, suspended, solubilized, and formulated into a formable and hardenable carrier material, in a physical-chemical form acceptable for administration by injection into an air-filled skull cavity as indicated herein.

The term "device" refers to the formulation as defined above after it has been injected and has hardened. Typical examples of such "devices" are stents, packings etc. A device of the present invention is typically non-metallic. The term "device" is used herein in its art-recognised meaning and refers to a spacer or spacing device suitably designed to fit, preferably in self-retaining manner, in a sinus of a patient. The term "device" as used herein includes reference to an instrument, implement, contrivance, implant, in vitro reagent, or other similar or related article, including any component, part, or accessory, which is intended for use in the diagnosis of disease or other conditions, or in the cure, mitigation, treatment, or prevention of disease, in a skull cavity.

The terms "hardenable", "hardening" and "hardened" refer respectively to the capability, the process, and the result of setting, curing or in general phase transition of a material or substance from a (shapeless) injectable formulation having a formable shape or low viscosity, to a device having a permanent shape or higher viscosity. For instance, a low viscosity gel may harden or stiffen into a more viscous gel structure following insertion and contact with the nasal/sinus tissues.

The term "permanent" is used to describe the state of the formulation once it has hardened from the gel state. In principle, the "permanent" shape is reached upon the formulation contacting the tissues of the cavity in which the formulation is injected. Typically, the "permanent" shape is enduring when a biostable material for the formulation is used, whereas the "permanent" shape is temporary when a biodegradable material is used.

The terms "individual" and "patient" for the purposes of the present invention refer to vertebrates, including mammals, birds and other animals, particularly humans. Thus the methods are applicable to both human therapy and veterinary applications. In preferred embodiments the individual or patient is a mammal, preferably a primate, and in most preferred embodiments the individual or patient is a human.

The terms "active ingredient", "active substance" or "drug", as used interchangeably herein mean any pharmaceutically active compound, substance or product, comprising both chemical entities and biotech/biological products, and include, but are not limited to, entities and products such as wound-healing substances, MMP inhibitors, COX-2 inhibitors, ACE inhibitors and ARBs, chymase inhibitors, therapeutic polymers, antibiotics, antiviral substances, peptides, proteins, growth factors and vaccines. Preferred active ingredients used in aspects of the present invention are those pharmaceutically active compounds that have a direct (MMP inhibitors) or indirect (COX-2, ACE inhibitors, ARBs etc) effect on inhibiting or reducing the activity of MMPs.

In principle, the active ingredient may be contacted with the formulation, or with any ingredient materials mixture used for preparing the formulation, in the form of a liquid such as a solution, a suspension, or a dispersion. Liquid active ingredient may be in oily or aqueous form or in the form of an emulsion such as a cream or paste; an active ingredient may also be a solid, such as a colloid or powder; or a semi-solid, such as a gel.

Preferred wound-healing active substances are substances that inhibit MMPs.

The term "medicament" means, in its broadest sense, a substance, formulation or device that treats or prevents or alleviates the symptoms of disease or condition in a patient to whom the medicament is administered. A medicament may be a prescription or non-prescription pharmaceutical preparation

The term "therapeutically effective amount" as used herein refers to an amount or dose of a therapeutic substance, *viz*. an MMP-inhibiting substance, that exerts a detectable therapeutic effect, *viz.* that improves the healing of wounds to the mucosa of the nasal sinus, in particular after sinus surgery, such as may be performed, by for instance FESS, in relation to complications of acute sinusitis, CRS and/or NP. The term "improve the healing of wounds" is to be understood as an improvement in time or quality of the wound healing including the prevention and/or reduction in the occurrence of abnormal scarring, super-infection, and fibrosis formation of such wounds as well as curing diseases and healing damage to affected sinus mucosal tissues. The therapeutic effect can be detected by, for example, imaging or direct observation of mucosal linings of sinuses treated by a method as herein described or contacted with a device as herein described by, for instance, endoscopic imaging techniques or by any other suitable method of assessing the progress or severity of sinusitis and sinus mucosal tissue wounds. The precise effective amount for any patient will depend upon the patient's age, body weight, general health, sex, diet, time of administration, drug interaction, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician or experimenter. Methods that permit the clinician to establish initial dosages are known in the art. The dosages determined for administration must be safe and efficacious. The exact dose will depend on the purpose of the treatment, and will be ascertainable by one skilled in the art using known techniques.

### B. The formulation

The formulation of the present invention is injectable. The advantage of this property is that it can easily be introduced at the desired location in a cavity of a vertebrate's skull, such as the paranasal sinus or nasal passageway.

Moreover, a formulation of an embodiment of the present invention comprises a formable material designed to adapt and conform to the shape of at least a part of a cavity of an individual's skull, preferably of a paranasal sinus and/or nasal passageway of a patient when the formulation is engaged therewith. The shape of the formulation after injection adapts to the anatomy of the specific cavity and acquires and maintains intimate contact with the walls of the cavity where it is deployed. As a result, the hardened formulation, or device, has a large effective area over which it is capable of directly releasing the active ingredient to the tissue that lines these walls. The device that results from hardening of the formulation is not intended for a particular sinus of a particular individual, but will fit in principle all sinuses of all individuals.

A formulation of an embodiment of the present invention is hardenable upon engagement with at least a part of a cavity of an individual's skull, preferably with at least a part of a paranasal sinus and/or nasal passageway of an individual, in particular the inner walls of the cavity, to provide said formulation with a permanent shape, thereby forming a device as herein described. Once in place, the formulation adapts to the shape of the sinus or passageway and is hardened in that shape and position. Upon hardening of the formulation, the resulting device is self-holding through the acquired specific (anatomical) shape. In one embodiment, the shape of the hardened device, such as a stent or packing, is such that it fits and is retained due to its shape in a particular part of the cavity, such as the ethmoid sinus and/or frontal sinus, and leaves room for airflow and also for drainage of mucous and/or wound fluid.

The formulation of the present invention is hardenable due to the presence therein of a formable and hardenable material as herein described. This material serves as main structural material for the device herein described and also as a matrix or carrier material for the active substance. The hardenability of the material is a virtue of its specific chemical structure. The material may be hardened by any method available, such as by molecular cross-linking.

In this embodiment, once hardened, the formulation produces the device. The hardened device may be solid or porous and may in principle take any one of a number of different forms such as in the form of a stent, a block, a foam, a sponge, a sheet, a sheath, a tube, coagulated granules or particles, a coating or a paving. In one embodiment, the hardened device has the form of a gel or soft solid. It is to be noted that a hardened device in the form of a gel or soft solid will generally have a higher viscosity than the corresponding formulation from which it originated.

The formulation may for instance consist of or comprise combined organic/inorganic materials and may be from various origin, natural, biological or synthetic. As suitable formable materials, both organic and inorganic materials, as well as combinations thereof may be used. Organic materials may be of non-polymeric or polymeric nature. Non-polymeric materials include non-water soluble materials such as sucrose acetate isobutyrate. Polymers provide for very suitable device materials. Polymers used in this embodiment of the invention can have linear, branched and dendritic structures, or can be interpenetrating networks. These polymers include the ability to tailor mechanical properties and degradation kinetics to suit various applications. Polymers can also be fabricated into various shapes. Numerous synthetic and natural or modified natural polymers can be used to prepare formulations useful in embodiments of the invention.

Representative synthetic polymers may include alkyl cellulose, cellulose esters, cellulose ethers, hydroxyalkyl celluloses, nitrocelluloses, polyalkylene glycols, polyalkylene oxides, polyalkylene terephthalates, polyalkylenes, polyamides, polyanhydrides, polycarbonates, polyesters, polyglycolides, polymers of acrylic and methacrylic esters, polyacrylamides, polyorthoesters, polyphosphazenes, polysiloxanes, polyurethanes, polyvinyl alcohols, polyvinyl esters, polyvinyl ethers, polyvinyl halides, polyvinylpyrrolidone, poly(ether ether ketone)s, silicone-based polymers and blends and copolymers of the above. The formulation may comprise both oligomers and polymers of the above.

Specific examples of these broad classes of polymers include poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), polyethylene, polypropylene, poly(ethylene glycol), poly(ethylene oxide), poly(propylene oxide), poly(ethylene terephthalate), poly(vinyl alcohols), poly(vinyl acetate), poly(vinyl chloride), polystyrene, polyurethane, poly(lactic acid), poly(butyric acid), poly(valeric acid), poly[lactide-co-glycolide], poly(fumaric acid), poly(maleic acid), copolymers of poly (caprolactone) or poly (lactic acid) with polyethylene glycol as well as copolymers and blends thereof.

The polymers used in devices may be non-biodegradable. The consequence thereof is that, after some time, the device must be removed. Examples of non-biodegradable polymers include poly(ethylene vinyl acetate)s, poly(meth)acrylic acids, polyamides, silicone-based polymers and copolymers and mixtures thereof. Another embodiment, the formable and hardenable device material is an injectable gel system with drug entrapped or covalently or physically bonded. The gel system may be a reactive self-forming gel hardenable by photopolymerization or electrophilic-neutrophilic polymerization, or an in situ-forming gel as herein described.

Suitable injectable formulations of one embodiment of the invention are for instance prepared from hydrogels. A hydrogel is a network of hydrophilic polymers that can swell in water and hold a large amount of water while maintaining the structure. A three-dimensional network is formed by crosslinking polymer chains. Crosslinking can be provided by covalent bonds, hydrogen bonding, van der Waals and ionic interactions, or physical entanglements. Hydrogels can protect the drug from hostile environments, e.g. the presence of enzymes, hydrogels can also control drug release by changing the gel structure in response to environmental stimuli. Hydrogels are called 'permanent' (or 'chemical') gels when these are covalently-crosslinked networks, and these are generated by crosslinking of water-soluble polymers. Gels may comprise hydrophobic materials, such as e.g. water-insoluble polymers or sucrose acetate isobutyrate, or copolymers with hydrophobic building blocks. In particular, introduction of hydrophobicity is important when it is required that the material reside for more than a couple of weeks and does not have quick drug release properties.

Photopolymerization is may be utilized for one embodiment of the present invention since it is capable of transforming a liquid macromer into a gel very rapidly, in a matter of seconds in most cases. In photopolymerization, an initiator is dissolved in a polymerizable precursor or its solution and exposed to a light source of appropriate wavelength. This converts the liquid into a gel state. Such photopolymerizations offer immense scope in biomaterials development because it offers a rapid, in situ processable technique to convert viscous liquids into solids. As an example, water-soluble precursors based on block copolymers of poly(ethylene glycol) (PEG) and poly(lactic acid) or poly(glycolic acid) with terminal acrylate groups can be used, and these precursors can be gelled in vivo by exposure to long wavelength ultraviolet light. The precursor may be photopolymerized from buffered saline solution while in contact with a tissue to be treated.

Other crosslinked polymers can be formed using electrophilic-nucleophilic reaction of polymers equipped with either electrophilic or nucleophilic functional groups. For example, a polyisocyanate or low molecular weight diisocyanate can be used as the electrophilic polymer or crosslinker, and a PEG with amine groups can be used as the nucleophilic precursor. Alternatively, one precursor has nucleophilic functional groups such as amines, whereas the other precursor has electrophilic functional groups such as N-hydroxysuccinimides. Thus, functional polymers such as proteins, poly(allyl amine), or amine-terminated di-or multifunctional PEG can be used. If it is desired that the biocompatible crosslinked polymer is biodegradable or absorbable, one or more precursors having biodegradable linkages present in between the functional groups may be used. In an embodiment, the active agent or agents are present in one of the precursors that are reacted to produce a crosslinked polymer network or gel.

Examples of biodegradable in situ forming drug delivery systems for local delivery of drugs are systems that form in situ upon exposure to a physiological condition in vivo, such as, e.g., temperature. Such biocompatible matrices are well known in the art and include, e.g., thermoplastic pastes (i.e., matrices that form upon cooling), and stereocomplexating materials.

Thermoplastic pastes include materials that have a melting temperature above body temperature, preferably between 25 and 65 °C, such as low molecular weight polymers or copolymers prepared from monomers such as D,L-lactide, glycolide, ε-caprolactone, trimethylene carbonate, dioxanone, ortho esters and poly(ethylene glycol) and modifications of these monomers, and blends of these (co)polymers. Stereocomplexation occurs e.g. by physical interaction between stereoisomers as *L*-lactate and *D*-lactate.

In another embodiment, the formable and hardenable device material is in the form of polymer microspheres dispersed in a hardenable gel. The gel may be injected in the target area (mucosal surface). Biodegradable microspheres can be prepared using any of the methods developed for making microspheres for drug delivery as described in literature, such as solvent evaporation, hot melt encapsulation, solvent removal, and spray drying. The selection of the method depends on the polymer selection, the size, external morphology, and crystallinity that are desired.

In solvent evaporation, the polymer is dissolved in a volatile organic solvent. The drug, either in soluble form or dispersed as fine particles, is added to the polymer solution, and the mixture is suspended in an aqueous phase that contains a surface active agent such as poly(vinyl alcohol). The resulting emulsion is stirred until most of the organic solvent evaporates, leaving solid microspheres and the resulting microspheres will be washed with water and dried overnight in a lyophilizer. Microspheres with different sizes (1-1000 microns) and morphologies can be obtained by this method.

In hot melt encapsulation, the polymer is first melted and then mixed with the solid particles of drug. The mixture is suspended in a non-miscible solvent such as silicone oil and, with continuous stirring, heated to 5 °C above the melting point of the polymer. Once the emulsion is stabilized, it is cooled until the polymer particles solidify. The resulting microspheres are washed by decantation with petroleum ether to give a free-flowing powder. Microspheres with diameters between 1 and 1000 microns can be obtained with this method. The external surface of spheres prepared with this technique is usually smooth and dense.

In the solvent removal method, the drug is dispersed or dissolved in a solution of a polymer in a volatile organic solvent like methylene chloride. The mixture is then suspended in oil, such as silicone oil, by stirring, to form an emulsion. Next, the solvent diffuses into the oil phase and the emulsion droplets harden into solid polymer microspheres. Unlike solvent evaporation, this method can be used to make microspheres from polymers with high melting points and a wide range of molecular weights. Microspheres having a diameter between 1 and 300 microns can be obtained with this procedure. The external morphology of the spheres is highly dependent on the type of polymer used.

In spray drying, the polymer is dissolved in a solvent like methylene chloride. A known amount of active drug is suspended (if insoluble) or co-dissolved (if soluble) in the polymer solution. The solution or the dispersion is then spray-dried. Microspheres ranging in diameter between 1 and 10 microns can be obtained with a morphology, which depends on the selection of polymer.

It is an embodiment of the present invention to use materials for manufacture of the device formulation, which are biodegradable after hardening, in which case the drug delivery device herein described comprises a biodegradable drug-comprising matrix. The biodegradable materials are suitably selected from the wide range of biodegradable polymers. The rate of degradation of the biodegradable device formed from the formulation is determined by factors such as configurational structure, copolymer ratio, crystallinity, molecular weight, morphology, stresses, amount of residual monomer, porosity and site of implantation. The skilled person will be able to choose the combination of factors and characteristics such that the rate of degradation is optimized.

Examples of biodegradable polymers include synthetic polymers such as polyesters, polyanhydrides, poly(ortho)esters, polyurethanes, siloxane-based polyurethanes, poly(butyric acid), tyrosine-based polycarbonates, and natural polymers and polymers derived therefrom such as albumin, alginate, casein, chitin, chitosan, collagen, dextran, elastin, proteoglycans, gelatin and other hydrophilic proteins, glutin, zein and other prolamines and hydrophobic proteins, starch and other polysaccharides including cellulose and derivatives thereof (e.g. methyl cellulose, ethyl cellulose, hydroxypropyl cellulose, hydroxypropyl methyl cellulose, hydroxybutyl methyl cellulose, carboxymethyl cellulose, cellulose acetate, cellulose propionate, cellulose acetate butyrate, cellulose acetate phthalate, cellulose acetate succinate, hydroxypropylmethylcellulose phthalate, cellulose triacetate, cellulose sulphate), polypeptides as poly-l-lysine and hybrid (synthetic/peptide)polymers, polyethylenimine, poly(allyl amine), polyglycosaminoglycans as polyhyaluronic acids, and combinations, copolymers, mixtures and chemical derivatives thereof (substitutions, additions of chemical groups, for example, alkyl, alkylene, hydroxylations, oxidations, and other modifications routinely made by those skilled in the art). In general, these materials degrade either by enzymatic hydrolysis or exposure to water *in vivo*, by surface or bulk erosion. The foregoing materials may be used alone, as physical mixtures (blends), or as a copolymer.

Other useful polymers are polyesters, polyanhydrides, poly(ortho)esters, and blends thereof. These polymers are advantageous due to their ease of degradation by hydrolysis of ester linkage, degradation products being resorbed through the metabolic pathways of the body in some cases and because of their potential to tailor the structure to alter degradation rates. The mechanical properties of the biodegradable material may be selected such that early degradation and concomitant loss of mechanical strength required for its functioning as a device is prevented.

Biodegradable polyesters are for instance poly(glycolic acid) (PGA), poly(lactic acid) (PLA), poly(lactic-co-glycolic acid) (PLGA), poly(dioxanone), poly(ε-caprolactone) (PCL), poly(3-hydroxybutyrate) (PHB), poly(3-hydroxyvalerate) (PHV), poly(lactide-co-caprolactone) (PLCL), poly(valerolactone) (PVL), poly(tartronic acid), poly(β-malonic acid), poly(propylene fumarate) (PPF), poly(ethylene glycol)/poly(lactic acid) (PELA) block copolymer, poly(L-lactic acid-ε-caprolactone) copolymer, and poly(lactide)-poly(ethylene glycol) copolymers.

Biodegradable polyanhydrides are for instance poly[1,6-bis(carboxyphenoxy)hexane], poly(fumaric-co-sebacic)acid or P(FA:SA), poly(sebacic acid-co-rinoleic acid) and such polyanhydrides may be used in the form of copolymers with polyimides or poly(anhydrides-co-imides) such as poly-[trimellitylimidoglycine-co-bis(carboxyphenoxy)hexane], poly[pyromellitylimidoalanine-co-1,6-bis(carbophenoxy)hexane], poly[sebacic acid-co-1,6-bis(*p*-carboxyphenoxy)hexane] or P(SA:CPH) and poly[sebacic acid-co-1,3-bis(*p*-carboxyphenoxy)propane] or P(SA:CPP).

### C. The active ingredient

The formulation of one embodiment of the present invention further comprises an active ingredient and is capable of and adapted for locally releasing, in a controlled manner over a predetermined period of time from 1 day to 12 months, a therapeutically effective amount of an MMP-inhibiting substance. By this it is meant that the device formed from the formulation locally releases medication in an appropriate concentration pattern over time.

Controlled release systems typically employ polymeric biomaterials in which the inhibiting substance is entrapped and released into the environment, with release typically occurring through a combination of surface desorption, diffusion and polymer degradation.

Controlled release relates to a release of the active ingredient over a predetermined period of time from 1 day to 12 months. The delivery of the medication is preferably substantially continuous, meaning that the delivery of drug occurs in a manner that is substantially uninterrupted for a pre-selected period of drug delivery and/or that the delivery of drug during that period occurs at a substantially constant, pre-selected rate (e.g., amount of drug per unit time).

The release profile for the active ingredient may comprise both immediate release, delayed or sustained release, the latter two are also termed "release in a controlled manner" herein.

Delayed release may be attained by matching the polymer used to the drug delivered or by providing an over-layer of a polymer that covers the layer or body containing the active ingredient, which over-layer must degrade prior to release of the active ingredient. Material may be same as the base product polymer or a separate biodegradable polymer.

The drug/active compound can be incorporated in microspheres, micelles or drug/polymer complexes with the aim of delay or target the release. These microspheres/micelles can be crosslinked forming a network by themselves or be dispersed in a gel as a composite system.

As stated, release of the active ingredient from the devices herein described may occur through drug diffusion, and/or polymer degradation, or a combination of these. For this purpose, the formulation may be produced from variety of natural and synthetic materials suitable for release of drugs, which can be categorized as either hydrophobic [e.g., poly(lactide-co-glycolide) (PLGA), polyanhydrides] or hydrophilic polymers [e.g., hyaluronic acid (HA), collagen, poly(ethylene glycol) (PEG)]. Synthetic polymers such as PLGA and polyanhydrides are very suitable for use in drug delivery applications of the present invention, as they are biocompatible and available in a range of copolymer ratios to control their degradation. Drug release from these polymers typically occurs through a combination of surface desorption, drug diffusion, and polymer degradation.

The formulation may be prepared from a material comprising an active ingredient such that the active ingredient is entrapped throughout the devices and is released therefrom.

The type of active substance to be delivered by the device formed from the formulation may comprise MMP inhibitors, e.g. doxycycline, TIMP-1, dexamethasone; COX-2 inhibitors; ACE-inhibitors and ARBs; Chymase inhibitors; therapeutic polymers, or a combination thereof.

The active substances used in the embodiments are those known to inhibit or block the production of proteins and enzymes known to be found in diseased nasal tissues, but which are not found, or are found at much lower concentrations, in healthy nasal tissues. Such proteins and enzymes are at least in part responsible for, or are initiators or catalysts of biochemical compounds responsible for the extensive remodeling resulting in the blockage in the paranasal sinus and/or nasal passageways. The active substances, when localized at the diseased location, work through various mechanisms; for example, some work by altering biochemical pathways that produce compounds that cause remodeling, others work by directly reducing the production of and/or occurrence of MMPs.

The presence of a bacterial and/or fungal infection in the host's tissue will initiate a host immune response in which MMPs play an important role. However, excess amounts or activity of these MMPs may cause severe tissue damage and favour dissemination and persistence of these organisms. Moreover, bacterial proteolytic enzymes may activate inactive MMP precursors, or proMMPs, into their active forms, which may further enhance tissue destruction. Some of the bacteria capable of secreting such proteolytic enzymes have been found in CRS patients. Therefore, reducing excess MMP activity can not only improve healing but also help to avoid recurrence of infection and eventually prevent the formation of a biofilm or reducing its detrimental effects. Herein, biofilms are broadly defined as adherent microorganisms within a polysaccharide matrix (so-called glycocalyx) and biofilm infection is responsible for both device-related and chronic infections. Without wishing to be bound by theory, it is believed that MMPs are a down-stream result of the body's response to a biofilm or that these microorganisms induce the activation of MMPs. Thus, the present invention also relates to the prevention of biofilm formation on devices or tissues in mucosa-lined cavities of an individual's skull, in particular in the (para)nasal cavities, or to reduce the detrimental effects of such biofilms on the mucosal tissue by providing the formulations, devices and therapeutic methods herein described. Moreover, the present invention relates to the eradication of biofilms by the performance of the devices described herein.

MMP inhibitors that may be used in aspects of the present invention include, but are not limited to, tetracycline and its derivatives, including but not limited to: natural tetracyclines, such as chlortetracycline, oxytetracycline, and tetracycline; semi-synthetic tetracyclines such as minocycline, doxycycline, and methacycline; and chemically modified tetracyclines ("CMTs"), such as CMT-1 (4-dedimethylamino-tetracycline), CMT-2 (tetracycline-nitrile), CMT-3 (6-demethyl-6-deoxy-4-dedimethylaminotetracycline); CMT-4 (7-chloro-4-dedimethylamino-tetracycline); CMT-5 (tetracyclinepyrazole); CMT-6 (4-dedimethylamino-4-hydroxytetracycline); CMT-7 (12-alpha-deoxy-4-dedimethylaminotetracycline); and CMT-8 (6 alpha deoxy-5 hydroxy-4-dedimethylaminotetracycline). Other MMP inhibitors which may be used according to the invention include but are not limited to hydroxamic acid; synthetic MMP inhibitors (which achieve inhibition through zinc-binding groups) including hydroxamate compounds, carboxylate compounds, aminocarboxylate compounds, sulphydryl compounds, phosphoric acid derivatives, mercaptoalcohols, and mercaptoketones, and the specific compounds Batimastat (BB-94; British Biotechnol.), Marimastat (BB-2516; British Biotechnol.), Ilomastat (GM6001; Glycomed), CT-1746 (Celltech), AG-3340 (Agouron), BAY 12-9566 (Bayer), CGS27023A (Novartis), D-5419 (Chiroscience), R0 32-3555 (Roche), G1168 (Glaxo Wellcome), G 1173 (Glaxo Wellcome) and CDP-845 (Celltech); and natural products that carry hydroxamic acid, including BE 16627B (Banyis), and Matlystatin B (Sankyo). Other suitable inhibitors include dexamethasone, oleic acid and chelating agents, such as EDTA.

Cyclooxygenase-2 (COX-2) is known to burn a fat in the body known as arachidonic acid (AA), a naturally occurring omega-6 fatty acid found in nearly all cell membranes in humans. Prostaglandin E2 (PGE2) is synthesized from the catalyzation of COX-2 and AA and, when PGE2 is taken up by macrophages, it results in MMP-9 formation. Thus, if any of COX-2, PGE2 or AA is suppressed, MMP-9 formation will be suppressed. Therefore, the present invention, in one aspect, provides COX-2 inhibitors as active substances. COX-2 inhibitors include Celecoxib, Rofecoxib, Valdecoxib, Etoricoxib, and Parecoxib, all of which are available in pharmacological preparations. Additionally, COX-2 inhibition has been demonstrated from herbs such as green tea, ginger, turmeric, chamomile, Chinese gold-thread, barberry, baikal skullcap, Japanese knotweed, rosemary, hops, feverfew, and oregano; and from other agents such as piroxican, mefenamic acid, meloxican, nimesulide, diclofenac, MF-tricyclide, raldecoxide, nambumetone, naproxen, herbimycin-A, and diaryl hydroxyfuranones.

Other therapeutic agents useful in embodiments of the present invention are angiotensin-converting enzyme (ACE) inhibitors and angiotensin receptor blockers (ARBs) that suppress the development of elastase-induced remodeling. Such ACE inhibitors known in the art are captopril, enalapril, losartan and lisinopril and the active forms of several ACE inhibitor prodrugs on the market. Known ARBs are losartan, valsartan, and telmisartan.

Chymase catalyzes the conversion of pro-MMP-9 to MMP-9, and chymase inhibitors such as NK3201 (Nippon Kayaku) can be useful in the present invention.

Alternatively, materials can be used that are not considered drugs, but that have a similar action (therapeutic polymers). In WO0056383 BF Goodrich Co. discloses sich polymeric compositions for reducing the activity of MMPs. These polymers are anionic polymers wherein the polymer is selected from the group of sulfonated and carboxylic acid plymers. Alternatively, Rimon Therapeutics owns a technology that is based on Therapeutic Polymers (Theramers™), which are advanced medical polymers that have biological activity of themselves, without the addition of pharmaceuticals. The MI Theramers™ are chemically derivatized hydroxamate-based polymers that contain groups that bind to zinc. As such these have the capability of inhibiting the zinc-dependent MMPs.

The range of loading of the drug to be delivered is typically between about 1% and 90%, depending on the form and size of the device to be delivered and on the target tissue.

### D. Therapeutic treatment

Described herein are devices for releasing active substances directly to damaged tissues in cavities of a vertebrate's skull, such as the paranasal sinus of a human patient and which have the advantage of providing direct and sufficient physical contact between the device and the tissue. Also described herein are the injectable formulations that can be hardened into the devices. Further described herein are methods for the preparation of these formulations and devices and to use as medicaments in (therapeutic) treatment and prevention of disease in mucosa-lined and air-filled cavities of a vertebrate's skull. The therapeutic treatment methods are suitable for treating any disorder in (para)nasal cavities or other skull cavities which may benefit from the direct administration of active substance to tissue in contact with the devices herein described, especially sinusitis.

Therapeutic treatment methods thus relate amongst others to the treatment of paranasal sinus disease, including treatment of sinusitis, and of chronic rhinosinusitis (CRS) and nasal polyposis (NP).

Disclosed herein is a method for treatment of a disease or damaged mucosal tissue in a paranasal sinus or nasal passageway of a patient comprises the steps of:
a) introducing into the paranasal sinus or nasal passageway of a patient an injectable formulation as herein described comprising an active substance and a formable material designed to adapt and conform to the shape of at least a part of said sinus or passageway when engaged therewith, wherein said material is hardenable upon engagement with at least a part of said sinus or passageway to provide a device with a permanent shape, and wherein said device is capable of releasing a therapeutically effective amount of said active substance when hardened, and
b) hardening said formulation.

The various embodiments of a suitable formulation are described as hereinabove for a formulation of the present invention.

Depending on the size and type of the device and the site of deployment, endoscopic techniques for the introduction of the formulation may be necessary. Such and other techniques are well within reach of the skilled person.

Generally, devices such as stents are inserted in a similar fashion regardless of the site or the disease being treated. Briefly, a pre-insertion examination, usually a diagnostic imaging procedure, endoscopy, or direct visualization at the time of surgery, is generally first performed in order to determine the appropriate positioning for stent insertion. The formulation of an embodiment of the present invention is capable of being deformed, so that they can be inserted through tiny cavities in for instance liquid form and then acquire a defined shape when inserted, i.e. when placed at the desired location.

Once in place and hardened, the device physically forces the walls of the passageway apart and holds them open. As such, they are capable of insertion via a small opening, and yet are still able to hold open a large diameter cavity or passageway.

Nasal devices such as stents are typically manoeuvred into place under direct visual control, taking particular care to place the stent precisely across the narrowing in the cavity being treated.

Since the treatment method herein described may even prevent the necessity of performing paranasal sinus surgery on said patient, this step is entirely optional. Details on the formulation and the introducing thereof into the paranasal sinus of the patient are as described above.

Therapeutic treatment methods herein described may also relate to the treatment of middle ear disease, including the treatment of acute and chronic middle ear infections (otitis media), cholesteatoma and middle ear adhesions.

A method herein described for treatment of a diseased or damaged (para)nasal mucosal tissue may also be applied to treatment of the middle ear tissues in a patient. Such a method comprises the steps of:
a) introducing into the middle ear cavity of said patient an injectable formulation as herein described comprising an active substance and a formable material designed to adapt and conform to the shape of at least a part of the middle ear cavity of a patient when engaged therewith, wherein said material is hardenable upon engagement with at least a part of said cavity to provide a device with a permanent shape and wherein said device is capable of releasing a therapeutically effective amount of said active substance when hardened, and
b) hardening said formulation

A higher viscosity or hardened shape is not as important in the middle ear as it is in the paranasal/sinus cavities. Biodegradability of the device is however preferred.

## Claims

1. An injectable formulation for use in treatment of a disease in a (para)nasal cavity in an individual's skull, the formulation comprising an active substance and a formable material designed to adapt and conform to the shape of at least a part of said cavity when engaged therewith, wherein said material has a formable shape or low viscosity and upon engagement with at least a part of said cavity is hardenable to provide a device with a permanent shape or higher viscosity, wherein said device when hardened provides controlled release over a predetermined period of time of from 1 day to 12 months of a therapeutically effective amount of said active substance, and said formable material:
a) is an injectable reactive self-forming gel system hardenable by photopolymerization or electrophilic-nucleophilic reaction, or an in-situ forming gel system hardenable by cooling or stereocomplexation, or
b) is in the form of polymer microspheres dispersed in a gel, or
c) comprises a water-soluble polymer which when engaged with at least a part of said cavity forms a hydrogel generated by crosslinking of the water-soluble polymer.

2. A formulation for use according to claim 1, wherein said device is biodegradable.

3. Formulation for use according to claim 1, wherein said formable material is a water-soluble polymer that forms a hydrogel generated by crosslinking.

4. Formulation for use according to claim 1, wherein said formable material is a reactive self-forming gel system.

5. Formulation for use according to claim 1, wherein said formable material is an in-situ forming gel system.

6. Formulation for use according to claim 1, wherein said formable material is in the form of polymer microspheres dispersed in a gel.

7. Formulation for use according to claim 1, wherein said formable material is biodegradable.

8. Formulation for use according to claim 1, wherein said active substance is selected from the group consisting of matrix metalloproteinase inhibitors, COX-2 inhibitors, ACE- inhibitors and ARBs, Chymase inhibitors, therapeutic polymers and combinations thereof.

9. Formulation for use according to claim 8, wherein said matrix metalloproteinase inhibitor is selected from doxycycline and dexamethasone.

10. Formulation for use according to claim 8, comprising at least one pharmaceutical agent involved in tissue remodelling processes in the paranasal sinus and/or nasal passageways.

11. An injectable formulation comprising an active substance and a formable material designed to adapt and conform to the shape of at least a part of a cavity of an individual's skull when engaged therewith, wherein said active substance is selected from the group consisting of matrix metalloproteinase inhibitors, COX-2 inhibitors, ACE- inhibitors and ARBs, Chymase inhibitors, therapeutic polymers and combinations thereof, wherein said material has a formable shape or low viscosity and upon engagement with at least a part of said cavity is hardenable to provide a device with a permanent shape or higher viscosity, wherein said device when hardened provides controlled release over a predetermined period of time of from 1 day to 12 months of a therapeutically effective amount of said active substance, and said formable material:
a) is an injectable reactive self-forming gel system hardenable by photopolymerization or electrophilic-nucleophilic reaction, or an in-situ forming gel system hardenable by cooling or stereocomplexation, or
b) is in the form of polymer microspheres dispersed in a gel, or
c) comprises a water-soluble polymer which when engaged with at least a part of said cavity forms a hydrogel generated by crosslinking of the water-soluble polymer.

12. Formulation according to claim 11, wherein said active substance comprises a matrix metalloproteinase inhibitor.

13. Formulation according to claim 11, wherein said formable material is an injectable reactive self-forming gel system hardenable by photopolymerization or electrophilic-nucleophilic reaction, or comprises a water-soluble polymer which when engaged with at least a part of said cavity forms a hydrogel generated by crosslinking of the water-soluble polymer.

14. Formulation according to claim 11, wherein said formable material is in the form of polymer microspheres dispersed in a gel, or an in-situ forming gel system hardenable by cooling or stereocomplexation.

## Patentansprüche

1. Injizierbare Formulierung zur Verwendung bei der Behandlung einer Erkrankung in einer (Para-) Nasenhöhle in dem Schädel eines Menschen, wobei die Formulierung einen Wirkstoff und ein verformbares Material, das ausgelegt ist, um sich an die Form von wenigstens einem Teil der Nasenhöhle bei Eingriff in dieselbe anzupassen und anzugleichen, umfasst, wobei das Material eine verformbare Form oder eine niedrige Viskosität aufweist und nach Eingriff in wenigstens einen Teil der Nasenhöhle aushärtbar ist, um eine Vorrichtung mit einer dauerhaften Form oder einer höheren Viskosität bereitzustellen, wobei die Vorrichtung nach Aushärten eine kontrollierte Freisetzung über einen vorbestimmten Zeitraum hinaus, von 1 Tag bis zu 12 Monaten, einer therapeutisch wirksamen Menge des Wirkstoffs bereitstellt, und das verformbare Material:
a) ein injizierbares, reaktives, selbstformendes Gelsystem ist, das durch Photopolymerisation oder elektrophile-nukleophile Reaktion aushärtbar ist, oder ein in situ formendes Gelsystem, das durch Abkühlen oder Stereokomplexierung aushärtbar ist, oder
b) in der Form von Polymermikrokügelchen besteht, die in einem Gel dispergiert sind, oder
c) ein wasserlösliches Polymer umfasst, das bei Eingriff in wenigstens einen Teil der Nasenhöhle ein Hydrogel bildet, das durch Vernetzen des wasserlöslichen Polymers erzeugt wird.

2. Formulierung zur Verwendung nach Anspruch 1, wobei die Vorrichtung biologisch abbaubar ist.

3. Formulierung zur Verwendung nach Anspruch 1, wobei das verformbare Material ein wasserlösliches Polymer ist, das ein Hydrogel bildet, das durch Vernetzen erzeugt wird.

4. Formulierung zur Verwendung nach Anspruch 1, wobei das verformbare Material ein reaktives, selbstformendes Gelsystem ist.

5. Formulierung zur Verwendung nach Anspruch 1, wobei das verformbare Material ein in situ formendes Gelsystem ist.

6. Formulierung zur Verwendung nach Anspruch 1, wobei das verformbare Material in der Form von Polymermikrokügelchen besteht, die in einem Gel dispergiert sind.

7. Formulierung zur Verwendung nach Anspruch 1, wobei das verformbare Material biologisch abbaubar ist.

8. Formulierung zur Verwendung nach Anspruch 1, wobei der Wirkstoff ausgewählt ist aus der Gruppe bestehend aus Matrix-Metallproteinase-Hemmern, COX-2-Hemmern, ACE-Hemmern und ARBs, Chymasehemmern, therapeutischen Polymeren und Kombinationen davon.

9. Formulierung zur Verwendung nach Anspruch 8, wobei der Matrix-Metallproteinase-Hemmer ausgewählt ist aus Doxycyclin und Dexamethason.

10. Formulierung zur Verwendung nach Anspruch 8, umfassend wenigstens ein Pharmazeutikum, das an den Geweberekonstruktionsprozessen in der Nasennebenhöhle und/oder den Nasenwegen beteiligt ist.

11. Injizierbare Formulierung, umfassend einen Wirkstoff und ein verformbares Material, das ausgelegt ist, um sich an die Form von wenigstens einem Teil der Nasenhöhle in dem Schädel eines Menschen bei Eingriff in dieselbe anzupassen und anzugleichen, wobei der Wirkstoff ausgewählt ist aus der Gruppe bestehend aus Matrix-Metallproteinase-Hemmern, COX-2-Hemmern, ACE-Hemmern und ARBs, Chymasehemmern, therapeutischen Polymeren und Kombinationen davon, wobei das Material eine verformbare Form oder eine niedrige Viskosität aufweist und nach Eingriff in wenigstens einen Teil der Nasenhöhle aushärtbar ist, um eine Vorrichtung mit einer dauerhaften Form oder einer höheren Viskosität bereitzustellen, wobei die Vorrichtung nach Aushärten eine kontrollierte Freisetzung über einen vorbestimmten Zeitraum hinaus, von 1 Tag bis zu 12 Monaten, einer therapeutisch wirksamen Menge des Wirkstoffs bereitstellt, und das verformbare Material:
a) ein injizierbares, reaktives, selbstformendes Gelsystem ist, das durch Photopolymerisation oder elektrophile-nukleophile Reaktion aushärtbar ist, oder ein in situ formendes Gelsystem, das durch Abkühlen oder Stereokomplexierung aushärtbar ist, oder
b) in der Form von Polymermikrokügelchen besteht, die in einem Gel dispergiert sind, oder
c) ein wasserlösliches Polymer umfasst, das bei Eingriff in wenigstens einen Teil der Nasenhöhle ein Hydrogel bildet, das durch Vernetzen des wasserlöslichen Polymers erzeugt wird.

12. Formulierung nach Anspruch 11, wobei der Wirkstoff einen Matrix-Metallproteinase-Hemmer umfasst.

13. Formulierung nach Anspruch 11, wobei das verformbare Material ein injizierbares, reaktives, selbstformendes Gelsystem ist, das durch Photopolymerisation oder elektrophile-nukleophile Reaktion aushärtbar ist, oder ein wasserlösliches Polymer umfasst, das bei Eingriff in wenigstens einen Teil der Nasenhöhle ein Hydrogel bildet, das durch Vernetzen des wasserlöslichen Polymers erzeugt wird.

14. Formulierung nach Anspruch 11, wobei das verformbare Material in der Form von Polymermikrokügelchen besteht, die in einem Gel dispergiert sind, oder ein in situ formendes Gelsystem ist, das durch Abkühlen oder Stereokomplexierung aushärtbar ist.

## Revendications

1. Formulation injectable destinée à être utilisée dans le traitement d'une maladie dans une cavité (para)nasale dans le crâne d'un individu, la formulation comprenant une substance active et un matériau façonnable conçu pour s'adapter et se conformer à la forme d'au moins une partie de ladite cavité lorsqu'il est introduit dedans, dans laquelle ledit matériau a une forme façonnable ou une faible viscosité et, après introduction dans au moins une partie de ladite cavité, est durcissable pour constituer un dispositif avec une forme permanente ou une viscosité plus élevée, ledit dispositif, lorsqu'il est durci, permettant une libération contrôlée pendant une période de temps prédéterminée allant de 1 jour à 12 mois d'une quantité thérapeutiquement efficace de ladite substance active, et ledit matériau façonnable :
a) étant un système de gel à auto-formation réactif injectable durcissable par photopolymérisation ou réaction électrophile-nucléophile, ou un système de gel se formant in situ durcissable par refroidissement ou stéréocomplexation, ou
b) se trouvant sous forme de microsphères polymères dispersées dans un gel, ou
c) comprenant un polymère hydrosoluble, qui, lorsqu'il est introduit dans au moins une partie de ladite cavité forme un hydrogel produit par réticulation du polymère hydrosoluble.

2. Formulation destinée à être utilisée selon la revendication 1, ledit dispositif étant biodégradable.

3. Formulation destinée à être utilisée selon la revendication 1, dans laquelle ledit matériau façonnable est un polymère hydrosoluble qui forme un hydrogel produit par réticulation.

4. Formulation destinée à être utilisée selon la revendication 1, dans laquelle ledit matériau façonnable est un système de gel à auto-formation réactif.

5. Formulation destinée à être utilisée selon la revendication 1, dans laquelle ledit matériau façonnable est un système de gel se formant in situ.

6. Formulation destinée à être utilisée selon la revendication 1, dans laquelle ledit matériau façonnable se trouve sous forme de microsphères polymères dispersées dans un gel.

7. Formulation destinée à être utilisée selon la revendication 1, dans laquelle ledit matériau façonnable est biodégradable.

8. Formulation destinée à être utilisée selon la revendication 1, dans laquelle ladite substance active est choisie dans le groupe consistant en inhibiteurs de métalloprotéinase de matrice, inhibiteurs de COX-2, inhibiteurs d'ACE et ARB, inhibiteurs de chymase, polymères thérapeutiques et combinaisons de ceux-ci.

9. Formulation destinée à être utilisée selon la revendication 8, dans laquelle ledit inhibiteur de métalloprotéinase de matrice est choisi parmi la doxycycline et la dexaméthasone.

10. Formulation destinée à être utilisée selon la revendication 8, comprenant au moins un agent pharmaceutique impliqué dans les processus de remodelage tissulaire dans le sinus paranasal et/ou les voies nasales.

11. Formulation injectable comprenant une substance active et un matériau façonnable conçu pour s'adapter et se conformer à la forme d'au moins une partie d'une cavité du crâne d'un individu lorsqu'il est introduit dedans, dans laquelle ladite substance active est choisie dans le groupe consistant en inhibiteurs de métalloprotéinase de matrice, inhibiteurs de COX-2, inhibiteurs d'ACE et ARB, inhibiteurs de chymase, polymères thérapeutiques et combinaisons de ceux-ci, dans laquelle ledit matériau a une forme façonnable ou une faible viscosité et, après introduction dans au moins une partie de ladite cavité, est durcissable pour constituer un dispositif avec une forme permanente ou une viscosité plus élevée, ledit dispositif, lorsqu'il est durci, permettant une libération contrôlée pendant une période de temps prédéterminée allant de 1 jour à 12 mois d'une quantité thérapeutiquement efficace de ladite substance active, et ledit matériau façonnable :
a) étant un système de gel à auto-formation réactif injectable durcissable par photopolymérisation ou réaction électrophile-nucléophile, ou un système de gel se formant in situ durcissable par refroidissement ou stéréocomplexation, ou
b) se trouvant sous forme de microsphères polymères dispersées dans un gel, ou
c) comprenant un polymère hydrosoluble, qui, lorsqu'il est introduit dans au moins une partie de ladite cavité forme un hydrogel produit par réticulation du polymère hydrosoluble.

12. Formulation selon la revendication 11, dans laquelle ladite substance active comprend un inhibiteur de métalloprotéinase de matrice.

13. Formulation selon la revendication 11, dans laquelle ledit matériau façonnable est un système de gel à auto-formation réactif injectable durcissable par photopolymérisation ou réaction électrophile-nucléophile, ou comprend un polymère hydrosoluble, qui, lorsqu'il est introduit dans au moins une partie de ladite cavité forme un hydrogel produit par réticulation du polymère hydrosoluble.

14. Formulation selon la revendication 11, dans laquelle ledit matériau façonnable se trouve sous forme de microsphères polymères dispersées dans un gel ou dans un système de gel se formant in situ durcissable par refroidissement ou stéréocomplexation.
